# FASCICULE DE BREVET EUROPEEN

(11) **EP 0 828 511 B1**
(45) Date de publication et mention de la délivrance du brevet: **24.10.2001**
(21) Numéro de dépôt: 96917554.6
(22) Date de dépôt: 24.05.1996
(51) Int. Cl.: A61K 39/385, A61K 39/39, A61K 39/102

(54) **Composition vaccinale anti-Haemophilus influenzae de type b comprenant du polyribosylribitol phosphate et son procédé de préparation**
Polyribosylribitol-phosphat enthaltende Haemophilus-influenzae-Typ-b-Impfstoffzubereitung und Verfahren zu deren Herstellung
Haemophilus influenzae type b vaccine composition containing polyribosylribitol phosphate and method for making the same

(30) Priorité: 24.05.1995 FR 9506417
(43) Date de publication de la demande: 18.03.1998
(73) Titulaire: Aventis Pasteur, 69367 Lyon Cedex 07 (FR)
(72) Inventeur: ARMINJON, François, F-69004 Lyon (FR); CARTIER, Jean-René, F-69005 Lyon (FR)
(74) Mandataire: Kerneis, Danièle
(86) Numéro de dépôt international: FR9600791
(87) Numéro de publication internationale: WO9637222

(56) Documents cités:
- EP-A- 0 101 562
- EP-A- 0 208 375
- EP-A- 0 320 942
- WO-A-93/24148
- Physicians' Desk Reference, 1992, Supplements for revision, pages 1237 and 1241
- Physicians' Desk Reference, 1995, pages 2363 and 2393

## Description

L'invention concerne le domaine des compositions vaccinales et plus particulièrement les compositions vaccinales comprenant au moins un antigène constitué par le polysaccharide capsulaire de Haemophilus influenzae type b ou polyribosylribitol phosphate à haut poids moléculaire couplé à l'anatoxine tétanique.

On connaît dans l'art antérieur, et notamment par l'article "Quantitative and Qualitative Analyses of Serum Antibodies Elicited in Adults by Haemophilus influenzae Type b and Pneumococcus Type 6A Capsular Polysaccharide Tetanus Toxoid Conjugates" Rachel Schneerson et al, Infect. Immun. May 1986, Volume 52, p.519-528, un antigène que l'on peut utiliser à des fins vaccinales chez l'homme afin de le protéger des infections provoquées par Haemophilus influenzae type b. Cet antigène est constitué par un polysaccharide capsulaire de la bactérie, le polyribosylribitol phosphate (ou PRP) qui est rendu T-dépendant grâce à un couplage à une protéine porteuse, l'anatoxine tétanique. Des essais réalisés chez des enfants rhésus ont montré, ainsi que le rapporte cet article, que la réponse immunitaire était à la fois plus importante et plus précoce, si l'antigène était associé à de l'hydroxyde d'aluminium. Cependant, ainsi que le mentionne un autre article intitulé "Clinical and Immunologic responses to the capsular polysaccharide of Haemophilus influenzae type b alone or conjugated to tetanus toxoid in 18-23 month-old children", Bo A. Claesson and al, The Journal of Pediatrics, May 1988, Volume 112, Number 5, p. 695 - 702, on a pu remarqué que cet antigène, adsorbé sur hydroxyde d'aluminium était, après stockage moins immunogène que l'antigène maintenu en solution saline, ce qui peut être dû à une dégradation du polysaccharide.

Afin de résoudre ce problème de stabilité des PRP-T, il a été proposé dans l'art antérieur de le lyophiliser. Cette solution, si elle permet bien à l'antigène de conserver son caractère immunogène au cours du temps, présente cependant des inconvénients, notamment au niveau de la fabrication ; la lyophilisation et les opérations particulières de conditionnement qu'elle requiert compliquent le procédé de fabrication, ce qui en augmente le coût. En outre, au moment de l'administration, il est nécessaire d'effectuer une reprise du lyophilisat, ce qui signifie qu'il est nécessaire de disposer en plus du lyophilisat d'un liquide de reprise de ce lyophilisât ; cette opération représente une contrainte supplémentaire pour le praticien et comporte, comme toute manipulation, le risque d'être mal effectuée.

En outre, un certain nombre de combinaisons vaccinales liquides possèdent des antigènes adsorbés sur un adjuvant à base d'aluminium et il serait avantageux de pouvoir, sans perte d'immunogénicité, leur ajouter l'antigène constitué par le PRP-T. En effet, la solution proposée par l'art antérieur et consistant en une seringue particulière à 2 compartiments (un premier compartiment contenant le PRP-T sous forme lyophilisée et un second compartiment contenant les autres antigènes en suspension aqueuse) dont le contenu est mélangé extemporanément au moment de l'administration n'est pas satisfaisante tant au niveau des coûts de fabrication qu'au niveau des opérations à effectuer par le praticien.

Il est donc souhaitable de pouvoir disposer de composition vaccinale liquide comprenant l'antigène constitué par le PRP-T ayant un très bon caractère immunogène conservé au cours du temps, et dont les conditions de fabrication permettent la production au moindre coût.

Pour atteindre ces buts, l'invention a pour objet une composition vaccinale comprenant au moins un antigène qui est le polyribosylribitol phosphate à haut poids moléculaire constitutif du polysaccharide capsulaire d'Haemophilus influenzae type b couplé à l'anatoxine tétanique, ainsi qu'un adjuvant à base d'aluminium, caractérisée en ce que l'adjuvant à base d'aluminium présente un point de charge zéro inférieur à 7,2.

On a ainsi remarqué que, de façon surprenante, dans ces conditions, le PRP-T conservait au cours du temps en milieu liquide, son très bon caractère immunogène.

Selon une caractéristique particulière de l'invention l'adjuvant à base d'aluminium comprend des hydroxydes d'aluminium auxquels ont été ajoutés des anions.

Ainsi, il est possible d'utiliser un adjuvant parfaitement qualifié pour une utilisation vaccinale tout en conservant au PRP-T en milieu liquide une très bonne immunogénicité.

Selon une autre caractéristique de l'invention, les anions sont choisis parmi les phosphates ou les citrates.

Ainsi, la composition obtenue présente toutes les garanties de sécurité nécessaires à une administration vaccinale.

Selon un mode de réalisation particulier de l'invention, la composition vaccinale comprend en outre une ou plusieurs des valences vaccinales choisies parmi : la diphtérie, le tétanos, la coqueluche, l'hépatite B, la poliomyélite.

On peut ainsi disposer de combinaisons vaccinales liquides, stables, dans lesquelles chaque antigène conserve son immunogénicité, ce qui permet au praticien, sans manipulation supplémentaire de sa part, de vacciner simultanément contre plusieurs maladies ; ceci permet de réduire les coûts à la fois en ce qui concerne les produits et en ce qui concerne le nombre de visites à effectuer.

L'invention a également pour objet un procédé de préparation d'une composition vaccinale comprenant au moins un antigène constitué par le polyribosylribitol phosphate à haut poids moléculaire constitutif du polysaccharide capsulaire de Haemophilus influenzae type b couplé à l'anatoxine tétanique, caractérisé en ce qu'il consiste à adjuver la compostion vaccinale au moyen d'une suspension de complexes d'aluminium ayant un point de charge zéro inférieur à 7,2.

La présente invention sera mieux comprise à la lecture de la description détaillée qui va suivre.

L'antigène constitué par le polysaccharide capsulaire de Haemophilus influenzae type b est un polymère linéaire consistant en ribose, ribitol et acide phosphorique dont la structure monomère est la suivante :

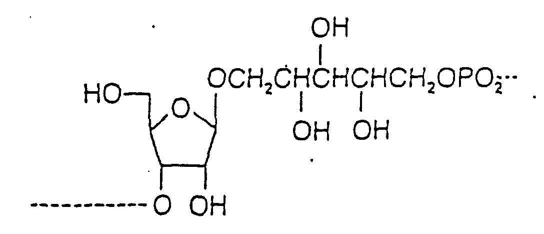

Le nombre de monomères de ce type est élevé (supérieur à 100), ce qui conduit à un polysaccharide dont le poids moléculaire est de l'ordre de 500 000 à 1 000 000.

Afin d'induire une réponse immunitaire des cellules T chez de jeunes enfants, cet antigène est conjugué à une protéine porteuse constituée par l'anatoxine tétanique.

Un tel antigène peut par exemple être obtenu selon la méthode décrite dans "Quantitative and Qualitative analysis of serum antibodies elicited in adults by Haemophilus influenzae type b and Pneumococcus type 6A capsular polysaccharide-tetanus toxoïd conjugates" Schneerson et al, Infect. Immun. 52 : 519 (1986).

Les caractéristiques propres à cet antigène : nombre élevé de monomères du polysaccharide, nature de la protéine porteuse, nature de la liaison entre le polysaccharide et la protéine porteuse, lui confèrent des qualités particulières et notamment une très bonne immunogénicité.

Afin de conserver ces qualités en milieu liquide au cours du temps, il a maintenant été trouvé qu'on pouvait utiliser des complexes d'aluminium dont le point de charge zéro était inférieur à 7,2. En effet, on a trouvé que, de façon surprenante, lorsque le PRP-T est associé à de tels complexes d'aluminium, son très bon caractère immunogène est conservé au cours du temps en milieu liquide et ce, quel que soit son degré de fixation aux complexes d'aluminium.

Le point de charge zéro des complexes d'aluminium est l'équivalent du point isoélectrique des protéines : c'est le pH auquel la charge en surface des complexes d'aluminium est nulle. En fait, ce point de charge zéro est approché par des mesures de potentiel zeta qui peuvent être réalisées selon différentes techniques, la méthode de base étant l'électrophorèse. Il est possible d'effectuer les mesures au moyen d'un appareil tel que le DELSA 440 de Coulters Electronics, Hialeah, Fl, USA.

Les méthodes et appareils de mesure pouvant être différents, les résultats obtenus peuvent également varier. Les complexes d'aluminium convenant aux fins de l'invention sont ceux pour lesquels ce point de charge zéro est inférieur à 7,2, cette valeur de 7,2 ne pouvant être qu'une valeur approximative.

Les complexes d'aluminium convenant aux fins de l'invention sont ceux qui, par nature, ont un point de charge zéro inférieur à 7,2 ou ceux qui peuvent être modifiés afin d'abaisser leur point de charge zéro.

Parmi les complexes d'aluminium ayant par nature un point de charge zéro inférieur à 7,2, on peut citer ceux communément appelés phosphates d'aluminium dans le domaine des adjuvants vaccinaux, même si d'un point de vue chimique, ils possèdent d'autres sels que les phosphates d'aluminium. Il s'agit par exemple du phosphate d'aluminium ADJUFOS® fourni par la société SUPERFOS BIOSECTOR a/s.

Il peut s'agir également de complexes d'aluminium obtenus par réaction de carbonate de sodium en tampon PBS sur des sulfates de potassium et d'aluminium.

Avec de tels complexes, bien que le PRP-T soit complètement ou partiellement lié aux complexes d'aluminium, son immunogénicité est conservée au cours du temps.

Alternativement, il est possible selon l'invention d'utiliser des complexes d'aluminium qui, par nature ont un point de charge zéro supérieur à 7,2 et qui sont modifiés pour abaisser ce point de charge.

Il s'agit notamment des complexes d'aluminium connus dans le domaine des adjuvants vaccinaux comme étant des hydroxydes d'aluminium, même si d'un point de vue chimique, ils ne sont pas constitués exclusivement d'hydroxyde d'aluminium. Il peut notamment s'agir de l'hydroxyde d'aluminium ALHYDROGEL ® fourni par la Société SUPERFOS BIOSECTOR a/s, du produit utilisé comme adjuvant dans le D.T. Coq ™ commercialisé par la Société PASTEUR MERIEUX S & V ou encore du produit utilisé comme adjuvant dans le Recombivax ® commercialisé par la Société MERCK.

Selon l'invention, la modification de ces complexes d'aluminium consiste en l'ajout d'anions. Les anions ajoutés peuvent être de différentes natures, à condition qu'ils présentent toutes les garanties de sécurité nécessaires pour une utilisation à des fins vaccinales. On a remarqué que l'ajout d'ions citrates ou d'ions phosphates convenait particulièrement bien aux fins de l'invention. Les ions phosphates peuvent notamment être apportés par une solution contenant du phosphate monopotassique, du phosphate disodique et du chlorure de sodium.

Il est possible également d'utiliser une combinaison de différents anions, par exemple une combinaison d'ions phosphates et d'ions carbonates.

Il est possible que les anions soient ajoutés à la suspension de complexes d'aluminium préalablement à l'ajout du PRP-T, ou que les anions soient ajoutés au PRP-T préalablement à sa mise en contact avec les complexes d'aluminium. Par commodité, on préfère mettre le PRP-T en suspension dans une solution contenant les anions choisis avant de le porter au contact de l'adjuvant.

La quantité d'anions ajoutés est calculée pour abaisser le point de charge zéro des complexes d'aluminium utilisés à une valeur inférieure à 7,2. Cette quantité varie donc en fonction de la nature des complexes d'aluminium utilisées ainsi que de la quantité d'anions éventuellement apportés par les substances tampon utilisées. Sa détermination est à la portée de l'homme de l'art.

Ainsi, on obtient une composition vaccinale stable à l'état liquide, c'est-à-dire dans laquelle le PRP-T conserve son bon caractère immunogène.

On a en outre remarqué que, grâce à l'ajout d'anions, la fixation du PRP-T aux complexes d'aluminium était réduite, ce qui doit contribuer au maintien de son intégrité et donc de son immunogénicité.

Bien que cela ne soit pas préférentiel au sens de l'invention, il est possible également d'ajouter des anions aux complexes d'aluminium ayant par nature un point de charge zéro déjà inférieur à 7,2. Dans ce cas, on a remarqué que le point de charge zéro peut être abaissé, et que la fixation du PRP-T aux complexes d'aluminium est également réduite.

Par fixation, on entend toute forme de liaison rendant le PRP-T inaccessible au dosage lorsque, après centrifugation, on recueille le surnageant.

Les compositions vaccinales selon l'invention comprennent l'antigène vaccinal constitué par le PRP-T mais peuvent comprendre également d'autres antigènes vaccinaux et notamment ceux destinés à protéger contre la diphtérie, le tétanos, la coqueluche (cellulaire ou acellulaire), la poliomyélite, l'hépatite A, l'hépatite B...etc. En fait, tout antigène vaccinal compatible avec le PRP-T et les complexes d'aluminium est susceptible d'entrer dans la composition vaccinale selon l'invention. On peut ainsi disposer de combinaisons vaccinales liquides permettant, en une seule administration, de vacciner contre plusieurs maladies. Les compositions vaccinales selon l'invention sont particulièrement adaptées à l'administration à de jeunes enfants.

De plus, on a remarqué que l'utilisation d'une telle composition vaccinale liquide permettait lors de la vaccination de rappel effectuée chez des nourrissons de 12 mois ayant reçu des injections à 2, 4 et 6 mois, d'accroître la production d'anticorps anti PRP-T de façon particulièrement accrue par rapport à un rappel effectué dans les mêmes conditions avec le vaccin TETRAct-HIB™ commercialisé par la Société PASTEUR MERIEUX Sérums et Vaccins.

### Exemple 1

On fabrique une composition vaccinale à partir des éléments suivants :
- anatoxine tétanique purifiée (ATP) 1 unité vaccinante
- anatoxine diphtérique purifiée (ADP) 1 unité vaccinante
- mélange cellulaire Pertussis 15 OU (unités opacimétriques)
- PRP-T (exprimé en poids de PRP) 12 µg
- merthiolate 43,75 µg
- hydroxyde d'aluminium (exprimé en Al) tel que celui présent dans le D.T. Coq ™ 0,3 mg
- phosphates 30 µmoles
- tampon tris 10 mmolaire comprenant du saccharose à 8,5 % 0,125 ml
- eau p.p.i. qsp 0,5 ml

Les ions phosphates sont apportés à partir d'une solution contenant du phosphate monopotassique, du phosphate disodique et du chlorure de sodium.

### Exemple 2

On a testé chez de jeunes enfants l'immunogénicité de la composition vaccinale obtenue selon l'exemple 1, comparativement à l'immunogénicité d'un vaccin de l'art antérieur, constitué par le vaccin commercialisé sous la marque TETRAct-HIB™ et qui possède les mêmes valences vaccinales mais où la valence PRP-T conservée lyophilisée est reconstituée juste avant l'injection par la composition vaccinale contenant les anatoxines diphtériques, tétaniques ainsi que le mélange cellulaire Pertussis.

Ce test a été réalisé sur un groupe de 262 nourrissons, dont 130 ont reçu la formulation selon l'exemple 1 et 132 ont reçu le vaccin commercial TETRAct-HIB ™. L'administration des vaccins a été effectuée à 2, 6 et 12 mois en infra-musculaire.

Avant immunisation, le titre GMT en anticorps anti-PRP était de 0,2 µg/ml dans les 2 groupes ; il était de 1,9 et 1,4 µg après la seconde injection et de 5,9 et 5,8 après la 3ème injection respectivement pour le vaccin selon l'invention et le vaccin de l'art antérieur.

Après la seconde injection, 98% (avec le vaccin selon l'invention) et 93 % (avec le vaccin selon l'art antérieur) des nourrissons avaient un taux d'anticorps anti-PRP supérieur à 0,15 µg/ml; ce taux était atteint après la 3ème injection chez 100 % des nourrissons ayant reçu le vaccin selon l'invention et chez 99 % des nourrissons ayant reçu le vaccin selon l'art antérieur.

Après la 3ème injection, les quantités d'anticorps dirigés contre chacune des valences vaccinales étaient en moyenne les suivantes :

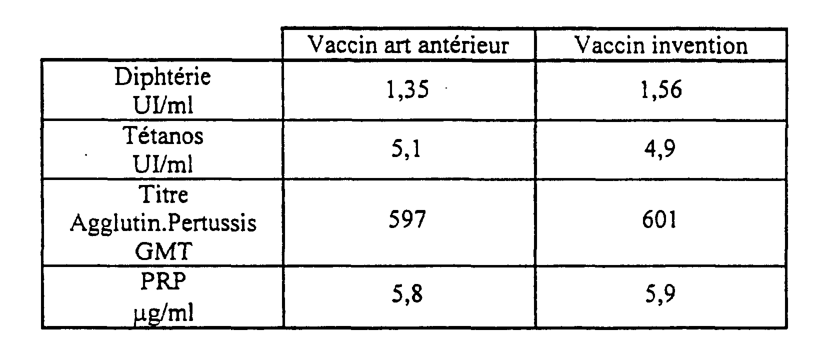

Après l'injection de rappel effectuée à 12 mois, les quantités d'anticorps étaient cette fois :

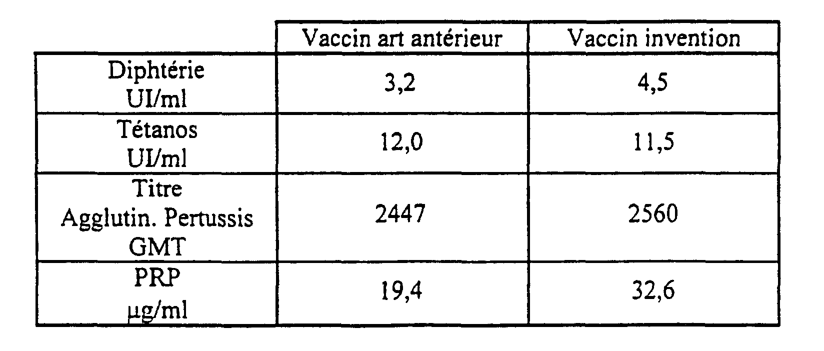

Ces résultats montrent que la combinaison vaccinale obtenue selon l'invention est stable ; en effet l'injection de rappel effectuée sur des enfants de 12 mois, a été réalisée avec un vaccin ayant été fabriqué 18 mois auparavant : or les résultats montrent que l'immunogénicité de chacun des antigènes de la combinaison est conservée. En outre, on obtient, de façon surprenante, un effet rappel nettement supérieur avec la composition vaccinale selon l'invention, par rapport à l'effet rappel obtenu avec un vaccin de l'art antérieur possédant les mêmes valences vaccinales.

### Exemple 3

Des doses de composition vaccinale telle que décrite à l'exemple 1 sont maintenues à + 4°C pendant 18 à 24 mois puis sont utilisées dans un essai clinique incluant 104 enfants.

Les titres obtenus pour chacune des valences vaccinales sont récapitulés dans le tableau ci-après.

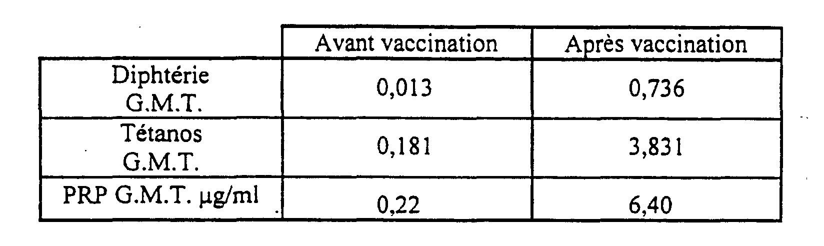

On peut ainsi voir que, même après un stockage de longue durée à +4°C, la composition vaccinale selon l'invention conserve son caractère immunogène, tant en ce qui concerne le PRP-T que les autres antigènes vaccinaux.

### Exemple 4

On prépare des compositions vaccinales comprenant chacune du PRP-T en une concentration de 20 µg de PRP/ml, en présence de complexes d'aluminium de différentes natures, et de différents points de charge zéro (PCZ). La quantité de complexes d'aluminium est telle que la concentration en aluminium dans la composition est de 0,6 g/l.

Composition 1 : Complexe d'aluminium constitué par de l'hydroxyde d'aluminium tel que celui utilisé dans le vaccin D.T. Coq™ commercialisé par PMsv. PCZ = 11,3

Composition 2 : Complexe d'aluminium constitué par de l'hydroxyde d'aluminium tel que celui utilisé dans le vaccin Recombivax ® commercialisé par Merck. PCZ = 7,4

Composition 3 : Complexe d'aluminium constitué par du phosphate d'aluminium obtenu par mélange de chlorure de sodium et de phosphate trisodique. PCZ = 6,2

Composition 4 : Complexe d'aluminium constitué par le produit appelé Alum et obtenu par réaction de carbonate de sodium en tampon PBS sur des sulfates de potassium et d'aluminium. PCZ = 5,4

Les compositions vaccinales sont obtenues par simple mélange des suspensions contenant les complexes d'aluminium et du PRP-T.

### Exemple 5

On teste l'immunogénicité chez des souris des différentes compositions obtenues.

Afin de vérifier la stabilité immunogénique, on soumet les compositions obtenues à des conditions de vieillissement accéléré, i.e. qu'on les conserve 2 semaines à 37°C.

Le test d'immunogénicité est effectué sur des souris de 22-24 g à qui on administre en sous-cutané des doses de 0,5 ml contenant chacune 2,5 µg de PRP. Les administrations sont effectuées à J0 et à J14. On prélève le sang des souris à J14 et à J21 et on détermine le taux d'anticorps par dosage radio immunologique. Le nombre de souris inoculées pour chaque composition vaccinale est de 8.

Le résultat est considéré satisfaisant si :
- on a au moins 75 % des souris à J21 qui ont un titre ≥ 0,5,
- on a une différence significative entre les résultats obtenus à J14 et ceux obtenus à J21.

On considère que la composition vaccinale obtenue est stable si les résultats obtenus après vieillissement accéléré sont satisfaisants.

Les résultats obtenus pour les compositions testées sont récapitulés dans le tableau ci-après :

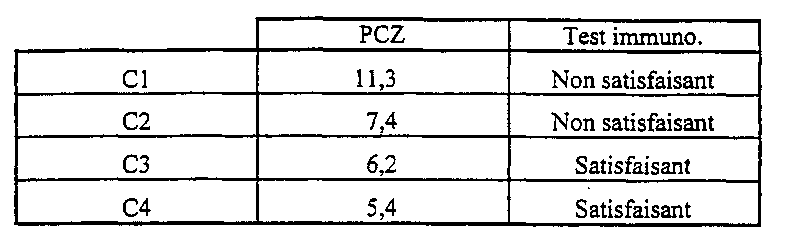

On voit ainsi que lorsque le point de charge zéro des complexes d'aluminium est un pH acide, la composition vaccinale obtenue est stable.

### Exemple 6

On vérifie pour chacune des compositions de l'exemple 4, le pourcentage de PRP-T fixé aux complexes d'aluminium.

Pour cela, on centrifuge chacune des compositions ; on recueille le surnageant dans lequel on dose par ELISA ou par RIA la quantité de PRP-T non fixé.

La différence entre la concentration de PRP-T dans la composition de départ et la quantité dosée dans le surnageant permet de déterminer le pourcentage de PRP-T fixé.

Les résultats obtenus sont relatés ci-après :

| | |
|---|---|
| C1 | 100% |
| C2 | 100% |
| C3 | 100% |
| C4 | 70% |

### Exemple 7

On modifie la composition 1 en lui ajoutant des ions phosphates afin d'obtenir une concentration de 50 mMole/l. Le test d'immunogénicité réalisé chez la souris après vieillissement accéléré de la solution conduit alors à un résultat satisfaisant.

La détermination du pourcentage de fixation de PRP-T aux complexes d'aluminium montre que, dans ces conditions, seul 20 % du PRP-T est fixé.

### Exemple 8

On modifie la composition 1 en lui ajoutant cette fois des ions citrates afin d'obtenir une concentration de 200 mMole/l.

Le test d'immunogénicité réalisé chez la souris après vieillissement accéléré de la solution conduit alors à un résultat satisfaisant.

La détermination du pourcentage de fixation de PRP-T aux complexes d'aluminium montre que, dans ces conditions, le PRP-T n'est plus du tout fixé.

### Exemple 9

On modifie la composition 2 en lui ajoutant des ions phosphates afin d'obtenir une concentration de 20 mMole/l.

Le test d'immunogénicité réalisé chez la souris après vieillissement accéléré de la solution conduit alors à un résultat satisfaisant. La détermination du pourcentage de fixation de PRP-T aux complexes d'aluminium montre que, dans ces conditions, le PRP-T n'est plus du tout fixé.

### Exemple 10

On modifie la composition 3 en lui ajoutant des ions phosphates en différentes quantités et on détermine le pourcentage de fixation du PRP-T aux complexes d'aluminium.

Si la quantité d'ions phosphates ajoutée est telle que la concentration en phosphates dans la composition est de 2 mMole/l, le pourcentage de PRP-T fixé est réduit à 10 %.

Si la quantité d'ions phosphates ajoutée est telle que la concentration en phosphates dans la composition est de 4 mMole/l, le PRP-T n'est plus du tout fixé.

Les tests d'immunogénicité réalisés chez la souris après vieillissement accéléré de la solution sont satisfaisants.

### Exemple 11

On modifie la composition 4 en lui ajoutant des ions phosphates pour atteindre une concentration de 60 mMole/l.

La détermination du pourcentage de PRP-T fixé aux complexes d'aluminium montre que, dans ces conditions, seul 30 % du PRP-T est fixé. Le test d'immunogénicité réalisé chez la souris après vieillissement accéléré de la solution conduit à un résultat satisfaisant.

### Exemple 12

On prépare une composition vaccinale à partir des éléments suivants :
- Hydroxyde d'Aluminium (exprimé en Al) 0,25 mg
- PRP-T (exprimé en poids de PRP) 10 µg
- ADP 1 dose vaccinante
- ATP 1 dose vaccinante
- Phosphates 15 µMoles
- Antigènes Polio
   type I 40 U
   type II 8 U
   type III 32 U
- Anatoxine Pertussis 25 µg
- F-HA Pertussis 25 µg
- tampon tris 50 mmolaire 0,125 ml comprenant du saccharose à 42,5 %
- eau p.p.i. qsp 0,5 ml

Les tests d'immunogénicité relatifs au PRP-T réalisés chez la souris avec une solution ainsi préparée, ainsi qu'avec une solution stockée 1 mois à 37°C, une solution stockée 2 mois à 25°C, et une solution stockée 6 mois à 4°C, ont tous conduit à des résultats satisfaisants, ce qui montre la stabilité du PRP-T dans un tel environnement.

### Exemple 13

On prépare une composition vaccinale à partir des éléments suivants :
- Hydroxyde d'Aluminium (exprimé en Al) 0,3 mg
- PRP-T (exprimé en poids de PRP) 10µg
- ADP 1 dose vaccinante
- ATP 1 dose vaccinante
- Anatoxine Pertussis 25 µg
- F-HA 25 µg
- Protéine Hbs (telle que présente dans le vaccin GenHevac B PASTEUR®) 20 µg
- Antigènes Polio
   type 1 40 U
   type II 8 U
   type III 32 U
- phosphates 20 µMoles
- carbonates 5 µMoles
- tampon tris 50 mMolaire comprenant du saccharose à 42,5 % 0,125 ml
- eau p.p.i. qsp 0,5 ml

On vérifie la stabilité de la solution ainsi préparée en la soumettant 2 semaines à 37°C et en effectuant ensuite un test d'immunogénicité du PRP-T chez la souris ainsi que cela a été décrit à l'exemple 5.

Les résultats obtenus sont satisfaisants.

On réalise un test d'immunogénicité de la protéine Hbs ; ce test est réalisé chez la souris et consiste à doser les anticorps anti-Hbs par ELISA, puis à déterminer la Dose Effective 50 % qui doit, pour que le test soit considéré satisfaisant, être inférieure à 0,970 µg de protéine Hbs.

Les résultats obtenus avec la solution préparée comme indiqué ci-dessus et maintenue 2 semaines à 37°C avant d'être testée, ont été satisfaisants.

### Exemple 14

On prépare une composition vaccinale à partir des éléments suivants :
- Hydroxyde d'Aluminium (exprimé en Al) 0,3 mg
- PRP-T (exprimé en poids de PRP) 10 µg
- ADP 1 dose vaccinante
- ATP 1 dose vaccinante
- Anatoxine Pertussis 10 µg
- F-HA 5 µg
- Fimbriae 5 µg
- Pertactine 3 µg
- Protéine Hbs (telle que présente dans le vaccin GenHevac B PASTEUR ® ) 20 µg
- Antigènes Polio
   type I 40 U
   type II 8 U
   type III 32 U
- phosphates 20 µMoles
- carbonates 10 µMoles
- MgCl₂ 5 µMoles
- tampon tris 50 mMolaire comprenant du saccharose à 42,5 % 0,125 ml
- eau p.p.i. qsp 0,5 ml

Les tests d'immunogénicité relatifs au PRP-T réalisés ainsi que cela a été décrit à l'exemple 5, et ceux relatifs à la protéine Hbs réalisés ainsi que cela a été décrit à l'exemple 13, ont tous conduit à des résultats satisfaisants, montrant la stabilité de la composition vaccinale selon l'invention.

## Revendications

1. Composition vaccinale comprenant au moins un antigène qui est le polyribosylribitol phosphate à haut poids moléculaire constitutif du polysaccharide capsulaire d'Haemophilus influenzae type b couplé à l'anatoxine tétanique ainsi qu'un adjuvant à base d'aluminium, **caractérisée en ce que** l'adjuvant à base d'aluminium présente un point de charge zéro inférieur à 7,2.

2. Composition vaccinale selon la revendication 1, **caractérisée en ce que** l'adjuvant à base d'aluminium comprend des hydroxydes d'aluminium auxquels ont été ajoutés des anions.

3. Composition vaccinale selon la revendication 2, **caractérisée en ce que** les anions sont choisis parmi les phosphates et les citrates.

4. Composition vaccinale selon une des revendications précédentes, **caractérisée en ce que** l'adjuvant à base d'aluminium comprend des phosphates d'aluminium.

5. Composition vaccinale selon une des revendications précédentes, **caractérisée en ce que** l'adjuvant à base d'aluminium comprend des sulfates de potassium et d'aluminium.

6. Composition vaccinale selon une des revendications précédentes, **caractérisée en ce qu'**elle comprend en outre une ou plusieurs des valences vaccinales choisies parmi: la diphtérie, le tétanos, la coqueluche, l'hépatite B, la poliomyélite.

7. Procédé de préparation d'une composition vaccinale comprenant au moins un antigène constitué par le polyribosylribitol phosphate à haut poids moléculaire constitutif du polysaccharide capsulaire de Haemophilus influenzae type b couplé à l'anatoxine tétanique, **caractérisé en ce qu'**il consiste à adjuver la composition vaccinale au moyen d'une suspension de complexes d'aluminium ayant un point de charge zéro inférieur à 7,2.

8. Procédé selon la revendication 7, **caractérisé en ce que** la suspension de complexes d'aluminium comprend des anions choisis parmi les phosphates et les citrates.

## Patentansprüche

1. Impfstoffzusammensetzung, enthaltend mindestens ein Antigen, bei dem es sich um mit Tetanustoxoid gekoppeltes, das Kapselpolysaccharid von Haemophilus influenzae Typ b bildendes hochmolekulares Polyribosylribitolphosphat handelt, und einen Hilfsstoff auf Aluminium-Basis, **dadurch gekennzeichnet, daß** der Hilfsstoff auf Aluminium-Basis einen Ladungsnullpunkt unter 7,2 aufweist.

2. Impfstoffzusammensetzung nach Anspruch 1,
**dadurch gekennzeichnet, daß** der Hilfsstoff auf Aluminium-Basis mit Anionen versetzte Aluminium-hydroxide enthält.

3. Impfstoffzusammensetzung nach Anspruch 2,
**dadurch gekennzeichnet, daß** die Anionen unter Phosphaten und Citraten ausgewählt sind.

4. Impfstoffzusammensetzung nach einem der vorhergehenden Ansprüche, **dadurch gekennzeichnet, daß** der Hilfsstoff auf Aluminium-Basis Aluminiumphosphate enthält.

5. Impfstoffzusammensetzung nach einem der vorhergehenden Ansprüche, **dadurch gekennzeichnet, daß** der Hilfsstoff auf Aluminium-Basis Kalium- und Aluminiumsulfate enthält.

6. Impfstoffzusammensetzung nach einem der vorhergehenden Ansprüche, **dadurch gekennzeichnet, daß** sie außerdem auch noch eine oder mehrere, unter Diphterie, Tetanus, Keuchhusten, Hepatitis B und Poliomyelitis ausgewählte Impfstoffvalenzen enthält.

7. Verfahren zur Herstellung einer Impfstoffzusammensetzung, enthaltend mindestens ein Antigen, bei dem es sich um mit Tetanustoxoid gekoppeltes, das Kapselpolysaccharid von Haemophilus influenzae Typ b bildendes hochmolekulares Polyribosylribitolphosphat handelt, **dadurch gekennzeichnet, daß** man der Impfstoffzusammensetzung als Hilfsstoff eine Suspension von Aluminiumkomplexen mit einem Ladungsnullpunkt unter 7,2 zusetzt.

8. Verfahren nach Anspruch 7, **dadurch gekennzeichnet, daß** die Suspension von Aluminiumkomplexen unter Phosphaten und Citraten ausgewählte Anionen enthält.

## Claims

1. Vaccine composition comprising at least one antigen which is high molecular weight polyribosylribitol phosphate forming the capsular polysaccharide of Haemophilus influenzae type b coupled to tetanus anatoxin as well as an aluminium-based adjuvant, **characterized in that** the aluminium-based adjuvant has a point of zero charge of less than 7.2.

2. Vaccine composition according to Claim 1,
**characterized in that** the aluminium-based adjuvant comprises aluminium hydroxides to which anions have been added.

3. Vaccine composition according to Claim 2,
**characterized in that** the anions are chosen from amongst the phosphates and the citrates.

4. Vaccine composition according to one of the preceding claims, **characterized in that** the aluminium-based adjuvant comprises aluminium phosphates.

5. Vaccine composition according to one of the preceding claims, **characterized in that** the aluminium-based adjuvant comprises potassium and aluminium sulphates.

6. Vaccine composition according to one of the preceding claims, **characterized in that** it additionally comprises one or more of the vaccine valencies chosen from amongst: diphtheria, tetanus, whooping cough, hepatitis B and poliomyelitis.

7. Method of peparation of a vaccine composition comprising at least one antigen consisting of high molecular weight polyribosylribitol phosphate forming the capsular polysaccharide of Haemophilus influenzae type b coupled to tetanus anatoxin, **characterized in that** it consists in adding an adjuvant to the vaccine composition by means of a suspension of aluminium complexes having a point of zero charge of less than 7.2.

8. Method according to Claim 7, **characterized in that** the suspension of aluminium complexes comprises anions chosen from amongst the phosphates and the citrates.
